# EUROPEAN PATENT APPLICATION

(11) **EP 4 684 847 A1**
(43) Date of publication of application: **28.01.2026**
(21) Application number: 24832263.8
(22) Date of filing: 22.05.2024
(51) Int. Cl.: A63B 24/00, A63B 71/06, A63B 21/00, A63B 21/005

(54) **MESSAGE OUTPUT METHOD AND ELECTRONIC DEVICE FOR PERFORMING SAME METHOD**

(30) Priority: 30.06.2023 KR 20230084976
(71) Applicant: Samsung Electronics Co., Ltd., Suwon-si, Gyeonggi-do 16677 (KR)
(72) Inventor: PARK, Hyunsu, Suwon-si Gyeonggi-do 16677 (KR); HAN, Hoon, Suwon-si Gyeonggi-do 16677 (KR); LEE, Jihye, Suwon-si Gyeonggi-do 16677 (KR)
(74) Representative: Gulde & Partner
(86) International application number: PCT/KR2024/006907
(87) International publication number: WO 2025/005479

(57) **Abstract**

According to an embodiment, a message output method may comprise the operations of: on the basis of an exercise history of a user of an electronic device, determining whether an exercise suggestion is required for the user; if it is determined that an exercise suggestion is required, determining a first state corresponding to the state of the user; if it is determined that an exercise suggestion is required, determining a second state corresponding to the state of a wearable device connected to the electronic device; on the basis of the first state and the second state, determining whether the user is in a state in which it is possible to exercise using the wearable device; if the user is in a state in which it is possible to exercise using the wearable device, generating a message for an exercise to be suggested to the user, on the basis of at least one of the exercise history, the first state, and the second state; and outputting the message.

## Description

### TECHNICAL FIELD

The present application relates to a technology for controlling wearable devices.

### BACKGROUND ART

A change into aging societies has contributed to a growing number of people who experience inconvenience and pain from reduced muscular strength or joint problems due to aging. Thus, there is a growing interest in walking assist devices that enable elderly users or patients with reduced muscular strength or joint problems to walk with less effort.

### DISCLOSURE OF THE INVENTION

### TECHNICAL SOLUTIONS

According to an embodiment, a message output method performed by an electronic device may include determining whether an exercise suggestion is required for a user based on an exercise history of the user of the electronic device, when it is determined that the exercise suggestion is required, determining a first state corresponding to a state of the user, when it is determined that the exercise suggestion is required, determining a second state corresponding to a state of a wearable device connected to the electronic device, determining whether the user is capable of performing an exercise using the wearable device based on the first state and the second state, when the user is capable of performing the exercise using the wearable device, generating a message about an exercise to be suggested to the user based on at least one of the exercise history, the first state, and the second state, and outputting the message.

According to an embodiment, an electronic device may include a communication module configured to exchange data with an external device, at least one processor connected to the communication module, and memory storing instructions executable by the at least one processor, and the instructions, when executed by the at least one processor, may cause the electronic device to perform receiving a signal for activating a welcome mode of the electronic device from an external electronic device connected to the electronic device through the communication module, and activating the welcome mode by controlling at least one of left and right leg support frames, a lighting unit, or a sound output module of the electronic device based on an exercise to be suggested to the user of the electronic device indicated by the signal.

According to an embodiment, an electronic device may include a communication module configured to exchange data with an external device, at least one processor connected to the communication module, and memory storing instructions executable by the at least one processor, and the instructions, when executed by the at least one processor, may cause the electronic device to perform determining whether an exercise suggestion is required for a user based on an exercise history of the user of the electronic device, when it is determined that the exercise suggestion is required, determining a first state corresponding to a state of the user, when it is determined that the exercise suggestion is required, determining a second state corresponding to a state of a wearable device connected to the electronic device, determining whether the user is capable of performing an exercise using the wearable device based on the first state and the second state, when the user is capable of performing the exercise using the wearable device, generating a message about an exercise to be suggested to the user based on at least one of the exercise history, the first state, and the second state, and outputting the message.

### BRIEF DESCRIPTION OF DRAWINGS

FIG. 1 is a diagram illustrating an overview of a wearable device worn on a body of a user, according to an embodiment.
FIG. 2 is a diagram illustrating an exercise management system including a wearable device and an electronic device, according to an embodiment.
FIG. 3 is a rear schematic view of a wearable device, according to an embodiment.
FIG. 4 is a left side view of a wearable device, according to an embodiment.
FIGS. 5A and 5B are diagrams illustrating a configuration of a control system of a wearable device, according to an embodiment.
FIG. 6 is a diagram illustrating an interaction between a wearable device and an electronic device, according to an embodiment.
FIG. 7 is a diagram illustrating a configuration of an electronic device, according to an embodiment.
FIG. 8 is a flowchart of a message output method according to an embodiment.
FIG. 9 is a flowchart of a method of generating a message according to an embodiment.
FIG. 10 is a diagram illustrating types of message outputs according to an embodiment.
FIG. 11 is a flowchart of a method of controlling a wearable device according to an embodiment.
FIGS. 12A and 12B are diagrams illustrating a welcome mode of a wearable device according to an embodiment.
FIGS. 13A and 13B are diagrams illustrating a welcome mode of a wearable device according to an embodiment.
FIG. 14 is a flowchart of a method of activating a welcome mode of a wearable device according to an embodiment.

### BEST MODE FOR CARRYING OUT THE INVENTION

Hereinafter, various embodiments of the present disclosure will be described with reference to the accompanying drawings. However, this is not intended to limit the present disclosure to specific embodiments, and it should be understood that various modifications, equivalents, and/or alternatives of the embodiments of the present disclosure are included.

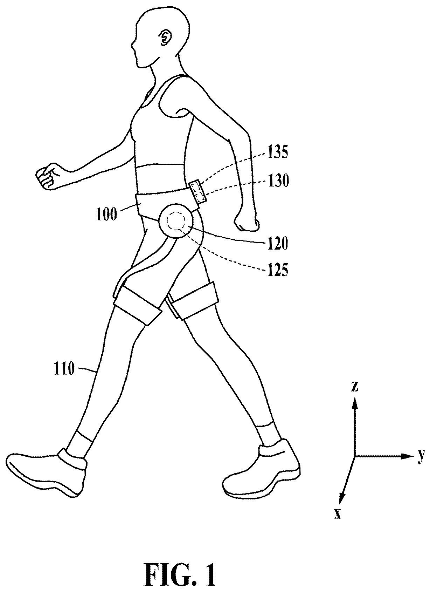

FIG. 1 is a diagram illustrating an overview of a wearable device worn on a body of a user, according to an embodiment.

Referring to FIG. 1, in an embodiment, a wearable device 100 may be a device worn on a body of a user 110 to assist the user 110 in walking, exercising, and/or working. In an embodiment, the wearable device 100 may be used to measure a physical ability (e.g., a walking ability, an exercise ability, or an exercise posture) of the user 110. In embodiments, the term "wearable device" may be replaced with "wearable robot," "walking assistance device," or "exercise assistance device." The user 110 may be a human or an animal, but is not limited thereto. The wearable device 100 may be worn on a body (e.g., a lower body (the legs, ankles, knees, etc.), an upper body (the torso, arms, wrists, etc.), or the waist) of the user 110 to apply an external force such as an assistance force and/or a resistance force to a body motion of the user 110. The assistance force may be a force applied in the same direction as the body motion direction of the user 110, the force to assist a body motion of the user 110. The resistance force may be a force applied in a direction opposite to the body motion direction of the user 110, the force hindering a body motion of the user 110. The term "resistance force" may also be referred to as "exercise load."

In an embodiment, the wearable device 100 may operate in a walking assistance mode for assisting the user 110 in walking. In the walking assistance mode, the wearable device 100 may assist the user 110 in walking by applying an assistance force generated by a driving module 120 of the wearable device 100 to the body of the user 110. The wearable device 100 may enable the user 110 to walk independently or to walk for a long time by providing a force required for the user 110 to walk, thereby expanding the walking ability of the user 110. The wearable device 100 may help in improving an abnormal walking habit or gait posture of a walker.

In an embodiment, the wearable device 100 may operate in an exercise assistance mode for enhancing the exercise effect of the user 110. In the exercise assistance mode, the wearable device 100 may impede a body movement of the user 110 or provide resistance to the body movement of the user 110 by applying a resistance force generated from the driving module 120 to the body of the user 110. When the wearable device 100 is a hip-type wearable device that is worn on a waist (or pelvis) and legs (e.g., thighs) of the user 110, the wearable device 100 may provide an exercise load to a leg motion of the user 110 while being worn on the legs, thereby enhancing the exercise effect on the legs of the user 110. In an embodiment, the wearable device 100 may apply an assistance force to the body of the user 110 to assist the user 110 in exercising. For example, when a person with a disability or an elderly person wants to exercise by wearing the wearable device 100, the wearable device 100 may provide an assistance force to assist a body motion during an exercise process. In an embodiment, the wearable device 100 may provide an assistance force and a resistance force in combination for each exercise section or time section, in such a manner of providing an assistance force in some exercise sections and a resistance force in other exercise sections.

In an embodiment, the wearable device 100 may operate in a physical ability measurement mode for measuring a physical ability of the user 110. The wearable device 100 may measure motion information of a user using sensors (e.g., an angle sensor 125 and an inertial measurement unit (IMU) 135) provided in the wearable device 100 while the user is walking or exercising, and evaluate the physical ability of the user based on the measured motion information. For example, a gait index or an exercise ability indicator (e.g., muscular strength, endurance, balance, or exercise motion) of the user 110 may be estimated through the motion information of the user 110 measured by the wearable device 100. The physical ability measurement mode may include an exercise posture measurement mode for measuring an exercise posture of a user.

In embodiments of the present disclosure, for convenience of description, the wearable device 100 is described as an example of a hip-type wearable device, as illustrated in FIG. 1, but the embodiments are not limited thereto. As described above, the wearable device 100 may be worn on body parts (e.g., upper arms, lower arms, hands, calves, and feet) other than the waist and legs (particularly, the thighs), and a shape and configuration of the wearable device 100 may vary depending on the body part on which the wearable device 100 is worn.

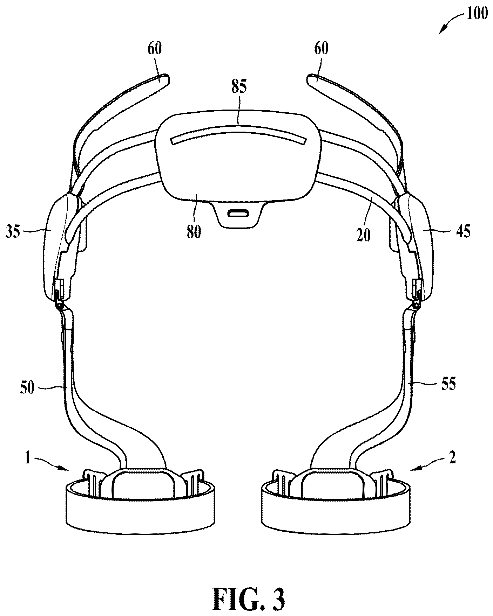

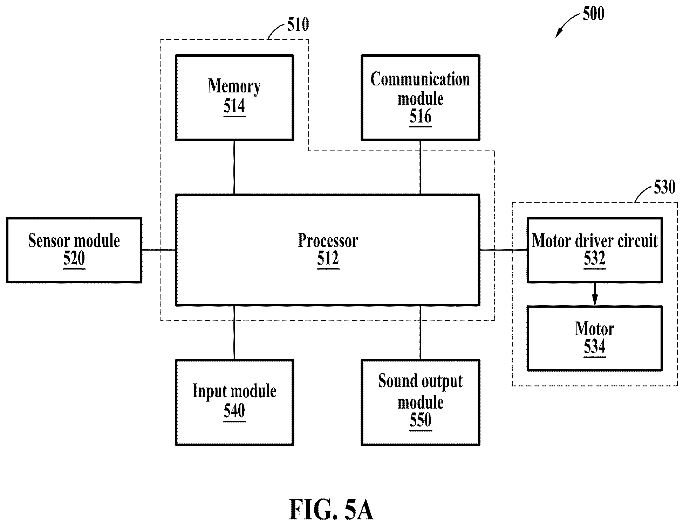

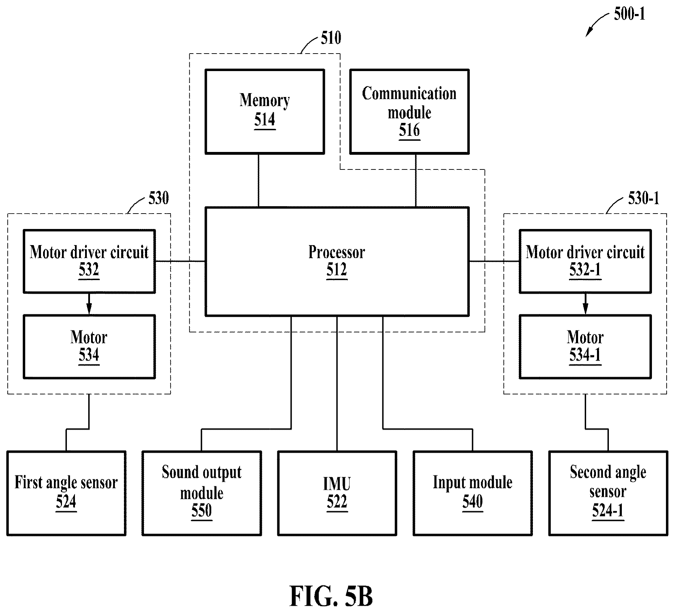

According to an embodiment, the wearable device 100 may include a support frame (e.g., leg support frames 50 and 55 and a waist support frame 20 of FIG. 3) configured to support the body of the user 110 when the wearable device 100 is worn on the body of the user 110, a sensor module (e.g., a sensor module 520 of FIG. 5A) configured to obtain sensor data including motion information on a body motion (e.g., a motion of a leg, and a motion of an upper body) of the user 110, the driving module 120 (e.g., driving modules 35 and 45 of FIG. 3) configured to generate torque to be applied to the legs of the user 110, and a control module 130 (e.g., a control module 510 of FIGS. 5A and 5B) configured to control the wearable device 100.

The sensor module may include the angle sensor 125 and the IMU 135. The angle sensor 125 may measure a rotation angle of a leg support frame of the wearable device 100 corresponding to a hip joint angle value of the user 110. The rotation angle of the leg support frame measured by the angle sensor 125 may be estimated as a hip joint angle value (or a leg angle value) of the user 110. The angle sensor 125 may include, for example, an encoder and/or a Hall sensor. In an embodiment, the angle sensor 125 may be present near each of a right hip joint and a left hip joint of the user 110. The IMU 135 may include an acceleration sensor and/or an angular velocity sensor, and may measure a change in acceleration and/or angular velocity according to a motion of the user 110. The IMU 135 may measure, for example, an upper body motion value of the user 110 corresponding to a motion value of a waist support frame (or a base body (a base body 80 of FIG. 3)) of the wearable device 100. A motion value of the waist support frame measured by the IMU 135 may be estimated as an upper body motion value of the user 110.

In an embodiment, the control module 130 and the IMU 135 may be arranged within the base body (e.g., the base body 80 of FIG. 3) of the wearable device 100. The base body may be disposed on a lumbar region (an area of the lower back) of the user 110 while the user 110 is wearing the wearable device 100. The base body may be formed on or attached to the outside of the waist support frame of the wearable device 100. The base body may be mounted on the lumbar region of the user 110 to provide a cushioning feeling to the lower back of the user 110 and may support the lower back of the user 110 together with the waist support frame.

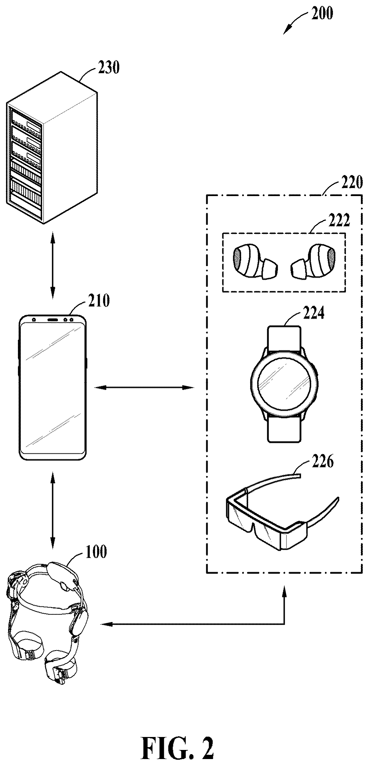

FIG. 2 is a diagram illustrating an exercise management system including a wearable device and an electronic device, according to an embodiment.

Referring to FIG. 2, a system 200 may include the wearable device 100 to be worn on a body of a user, an electronic device 210, another wearable device 220, and a server 230. In an embodiment, at least one (e.g., the other wearable device 220 or the server 230) of these devices may be omitted from the system 200, or one or more other devices (e.g., an exclusive controller device of the wearable device 100) may be added thereto.

In an embodiment, the wearable device 100 may be worn on the body of the user in a walking assistance mode to assist a motion of the user. For example, the wearable device 100 may be worn on legs of the user to help the user in walking by generating an assistance force for assisting a leg motion of the user.

In an embodiment, the wearable device 100 may generate a resistance force for hindering a body motion of the user or an assistance force for assisting a body motion of the user and apply the generated resistance force or assistance force to the body of the user to enhance the exercise effect of the user in an exercise assistance mode. In the exercise assistance mode, the user may select, through the electronic device 210, an exercise program (e.g., squat, split lunge, dumbbell squat, lunge and knee up, stretching, or the like) to perform using the wearable device 100 and/or an exercise intensity to be applied to the wearable device 100. The wearable device 100 may control a driving module of the wearable device 100 according to the exercise program selected by the user and obtain sensor data including motion information of the user through a sensor module. The wearable device 100 may adjust the strength of the resistance force or assistance force applied to the user according to the exercise intensity selected by the user. For example, the wearable device 100 may control the driving module to generate a resistance force corresponding to the exercise intensity selected by the user.

In an embodiment, the wearable device 100 may be used to measure a physical ability of the user by interworking with the electronic device 210. The wearable device 100 may operate in a physical ability measurement mode, which is a mode for measuring the physical ability of the user, under a control of the electronic device 210, and may transmit sensor data obtained by a motion of the user in the physical ability measurement mode to the electronic device 210. The electronic device 210 may estimate the physical ability of the user by analyzing the sensor data received from the wearable device 100.

The electronic device 210 may communicate with the wearable device 100 and may remotely control the wearable device 100 or provide the user with state information about a state (e.g., a booting state, a charging state, a sensing state, or an error state) of the wearable device 100. The electronic device 210 may receive sensor data obtained by a sensor of the wearable device 100 from the wearable device 100 and estimate the physical ability of the user or an exercise result based on the received sensor data. In an embodiment, when the user exercises wearing the wearable device 100, the wearable device 100 may obtain sensor data including motion information of the user using sensors and transmit the obtained sensor data to the electronic device 210. The electronic device 210 may extract a motion value of the user from the sensor data and evaluate an exercise posture of the user based on the extracted motion value. The electronic device 210 may provide the user with an exercise posture measured value and exercise posture evaluation information related to the exercise posture of the user through a graphical user interface (GUI).

In an embodiment, the electronic device 210 may execute a program (e.g., an application) configured to control the wearable device 100, and the user may adjust an operation or a set value of the wearable device 100 (e.g., the magnitude of torque output from a driving module (e.g., driving modules 35 and 45 of FIG. 3), the volume of audio output from a sound output module (e.g., a sound output module 550 of FIGS. 5A and 5B), or the brightness of a lighting unit (e.g., a lighting unit 85 of FIG. 3)) through the corresponding program. The program executed by the electronic device 210 may provide a GUI for interaction with the user. The electronic device 210 may be a device in various forms. For example, the electronic device 210 may include, but is not limited to, a portable communication device (e.g., a smartphone), a computer device, an access point, a portable multimedia device, or a home appliance device (e.g., a television, an audio device, a projector device).

According to an embodiment, the electronic device 210 may be connected to the server 230 using short-range wireless communication or cellular communication. The server 230 may receive user profile information of the user who uses the wearable device 100 from the electronic device 210 and store and manage the received user profile information. The user profile information may include, for example, information about at least one of a name, age, gender, height, weight, or body mass index (BMI). The server 230 may receive exercise history information about an exercise performed by the user from the electronic device 210 and store and manage the received exercise history information. The server 230 may provide the electronic device 210 with various exercise programs or physical ability measurement programs that may be provided to the user.

According to an embodiment, the wearable device 100 and/or the electronic device 210 may be connected to the other wearable device 220. The other wearable device 220 may include, for example, wireless earphones 222, a smartwatch 224, or smart glasses 226, but is not limited thereto. In an embodiment, the smartwatch 224 may measure a biosignal including heart rate information of the user and transmit the measured biosignal to the electronic device 210 and/or the wearable device 100. The electronic device 210 may estimate the heart rate information (e.g., a current heart rate, a maximum heart rate, and an average heart rate) of the user based on the biosignal received from the smartwatch 224 and provide the estimated heart rate information to the user.

In an embodiment, the exercise result information, physical ability information, and/or exercise posture evaluation information evaluated by the electronic device 210 may be transmitted to the other wearable device 220 and provided to the user through the other wearable device 220. State information of the wearable device 100 may also be transmitted to the other wearable device 220 and provided to the user through the other wearable device 220. In an embodiment, the wearable device 100, the electronic device 210, and the other wearable device 220 may be connected to each other through wireless communication (e.g., Bluetooth communication or wireless-fidelity (Wi-Fi) communication).

In an embodiment, the wearable device 100 may provide (or output) feedback (e.g., visual feedback, auditory feedback, or haptic feedback) corresponding to a state of the wearable device 100 according to a control signal received from the electronic device 210. For example, the wearable device 100 may provide visual feedback through the lighting unit (the lighting unit 85 of FIG. 3) and auditory feedback through the sound output module (e.g., the sound output module 550 of FIGS. 5A and 5B). The wearable device 100 may include a haptic module and provide haptic feedback in the form of vibration to the body of the user through the haptic module. The electronic device 210 may also provide (or output) feedback (e.g., visual feedback, auditory feedback, or haptic feedback) corresponding to the state of the wearable device 100.

In an embodiment, the electronic device 210 may present a personalized exercise goal to the user in the exercise assistance mode. The personalized exercise goal may include respective target amounts of exercise for exercise types (e.g., strength exercise, balance exercise, and aerobic exercise) desired by the user, determined by the electronic device 210 and/or the server 230. When the server 230 determines a target amount of exercise, the server 230 may transmit information about the determined target amount of exercise to the electronic device 210. The electronic device 210 may personalize and present the target amounts of exercise for the exercise types, such as strength exercise, aerobic exercise, and balance exercise, according to a desired exercise program (e.g., squat, split lunge, or a lunge and knee up) and/or physical characteristics (e.g., the age, height, weight, and BMI) of the user. The electronic device 210 may display a GUI screen displaying the target amounts of exercise for the respective exercise types on a display.

In an embodiment, the electronic device 210 and/or the server 230 may include a database in which information about a plurality of exercise programs to be provided to the user through the wearable device 100 is stored. To achieve an exercise goal of the user, the electronic device 210 and/or the server 230 may recommend an exercise program suitable for the user. The exercise goal may include, for example, at least one of muscle strength improvement, physical strength improvement, cardiovascular endurance improvement, core stability improvement, flexibility improvement, or symmetry improvement. The electronic device 210 and/or the server 230 may store and manage the exercise program performed by the user, results of performing the exercise program, and the like.

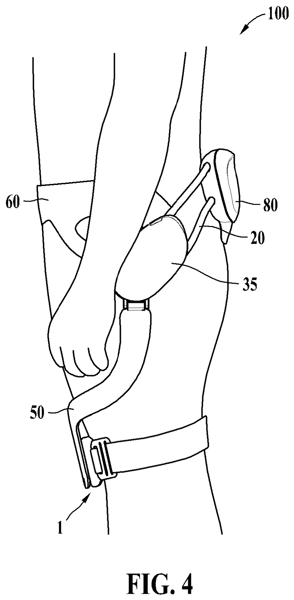

FIG. 3 is a rear schematic view of a wearable device, according to an embodiment. FIG. 4 is a left side view of a wearable device, according to an embodiment.

Referring to FIGS. 3 and 4, the wearable device 100 according to an embodiment may include the base body 80, the waist support frame 20, the driving modules 35 and 45, the leg support frames 50 and 55, thigh fastening portions 1 and 2, and a waist fastening portion 60. The base body 80 may include the lighting unit 85. In an embodiment, at least one (e.g., the lighting unit 85) of the above components may be omitted from the wearable device 100, or one or more other components (e.g., a haptic module) may be added to the wearable device 100.

The base body 80 may be on the waist of a user when the user wears the wearable device 100. The base body 80 worn on the waist of the user may cushion and support the waist of the user. The base body 80 may be hung on a hip region (an area of the hips) of the user such that the wearable device 100 may not be deviated downward due to gravity while the user is wearing the wearable device 100. The base body 80 may distribute a portion of the weight of the wearable device 100 to the lower back of the user while the user is wearing the wearable device 100. The base body 80 may be connected to the waist support frame 20. Waist support frame connecting elements (not shown) to be connected to the waist support frame 20 may be provided at both end portions of the base body 80.

In an embodiment, the lighting unit 85 may be arranged on an outer side of the base body 80. The lighting unit 85 may include a light source (e.g., a light-emitting diode (LED)). The lighting unit 85 may emit light under a control of a control module (not shown) (e.g., the control module 510 of FIGS. 5A and 5B). According to an embodiment, the control module may control the lighting unit 85 to provide (or output) visual feedback corresponding to the state of the wearable device 100 to the user through the lighting unit 85.

The waist support frame 20 may extend from both end portions of the base body 80. The lumbar region of the user may be accommodated inside the waist support frame 20. The waist support frame 20 may include at least one rigid body beam. Each beam may be in a curved shape having a preset curvature to enclose the lumbar region of the user. The waist fastening portion 60 may be connected to an end portion of the waist support frame 20. The driving modules 35 and 45 may be connected to the waist support frame 20.

In an embodiment, the control module, an IMU (not shown) (e.g., the IMU 135 of FIG. 1 or an IMU 522 of FIG. 5B), a communication module (not shown) (e.g., a communication module 516 of FIGS. 5A and 5B), and a battery (not shown) may be arranged inside the base body 80. The base body 80 may protect the control module, the IMU, the communication module, and the battery. The control module may generate a control signal for controlling an operation of the wearable device 100. The control module may include a control circuit including a processor configured to control actuators of the driving modules 35 and 45 and a memory. The control module may further include a power supply module (not shown) to supply power from a battery to each of the components of the wearable device 100.

In an embodiment, the wearable device 100 may include a sensor module (not shown) (e.g., the sensor module 520 of FIG. 5A) configured to obtain sensor data from at least one sensor. The sensor module may obtain sensor data that changes according to a motion of the user. In an embodiment, the sensor module may obtain sensor data including motion information of the user and/or motion information of the components of the wearable device 100. The sensor module may include, for example, an IMU (e.g., the IMU 135 of FIG. 1 or the IMU 522 of FIG. 5B) configured to measure an upper body motion value of the user or a motion value of the waist support frame 20, and an angle sensor (e.g., the angle sensor 125 of FIG. 1 or a first angle sensor 524 and a second angle sensor 524-1 of FIG. 5B) configured to measure a hip joint angle value of the user or a motion value of the leg support frames 50 and 55, but is not limited thereto. For example, the sensor module may further include at least one of a position sensor, a temperature sensor, a biosignal sensor, or a proximity sensor.

The waist fastening portion 60 may be connected to the waist support frame 20 to fasten the waist support frame 20 to the waist of the user. The waist fastening portion 60 may include, for example, a pair of belts.

The driving modules 35 and 45 may generate an external force (or torque) to be applied to the body of the user based on the control signal generated by the control module. For example, the driving modules 35 and 45 may generate an assistance force or resistance force to be applied to legs of the user. In an embodiment, the driving modules 35 and 45 may include a first driving module 45 disposed in a position corresponding to a position of a right hip joint of the user, and a second driving module 35 disposed in a position corresponding to a position of a left hip joint of the user. The first driving module 45 may include a first actuator and a first joint member, and the second driving module 35 may include a second actuator and a second joint member. The first actuator may provide power to be transmitted to the first joint member, and the second actuator may provide power to be transmitted to the second joint member. The first actuator and the second actuator may each include a motor configured to generate power (or a torque) by receiving electric power from the battery. When the motor is supplied with electric power and driven, the motor may generate a force (an assistance force) for assisting a body motion of the user or a force (a resistance force) for hindering a body motion of the user. In an embodiment, the control module may adjust the strength and direction of the force generated by the motor by adjusting the voltage and/or current supplied to the motor.

In an embodiment, the first joint member and the second joint member may receive power from the first actuator and the second actuator, respectively, and may apply an external force to the body of the user based on the received power. The first joint member and the second joint member may be arranged at positions corresponding to joint portions of the user, respectively. One side of the first joint member may be connected to the first actuator, and the other side of the first joint member may be connected to a first leg support frame 55. The first joint member may be rotated by the power received from the first actuator. An encoder or a Hall sensor that may operate as an angle sensor configured to measure the rotational angle of the first joint member (corresponding to the joint angle of the user) may be arranged on one side of the first joint member. One side of the second joint member may be connected to the second actuator, and the other side of the second joint member may be connected to a second leg support frame 50. The second joint member may rotate by the power relayed from the second actuator. An encoder or a Hall sensor that may operate as an angle sensor configured to measure a rotation angle of the second joint member may be arranged on one side of the second joint member.

In an embodiment, the first actuator may be arranged in a lateral direction of the first joint member, and the second actuator may be arranged in a lateral direction of the second joint member. A rotation axis of the first actuator and a rotation axis of the first joint member may be spaced apart from each other, and a rotation axis of the second actuator and a rotation axis of the second joint member may also be spaced apart from each other. However, embodiments are not limited thereto, and an actuator and a joint member may share a rotation axis. In an embodiment, each actuator may be spaced apart from a corresponding joint member. In this case, the driving module 35, 45 may further include a power transmission module (not shown) configured to transmit power from the actuator to the joint member. The power transmission module may be a rotary body, such as a gear, or a longitudinal member, such as a wire, a cable, a string, a spring, a belt, or a chain. However, the scope of the embodiment is not limited by a positional relationship between an actuator and a joint member and a power transmission structure described above.

In an embodiment, the leg support frame 50, 55 may support a leg (e.g., a thigh) of the user when the wearable device 100 is worn on the leg of the user. For example, the leg support frame 50, 55 may transmit power (a torque) generated by the driving module 35, 45 to the thigh of the user, and the power may function as an external force to be applied to a motion of the leg of the user. As one end portion of the leg support frame 50, 55 is connected to a joint member to rotate and the other end portion of the leg support frame 50, 55 is connected to the thigh fastening portion 1, 2, the leg support frame 50, 55 may transmit the power generated by the driving module 35, 45 to the thigh of the user while supporting the thigh of the user. For example, the leg support frame 50, 55 may push or pull the thigh of the user. The leg support frames 50 and 55 may extend in a longitudinal direction of the thighs of the user. The leg support frames 50 and 55 may be bent to surround at least a portion of the circumference of the thighs of the user. The leg support frames 50 and 55 may include the first leg support frame 55 configured to support the right leg of the user and the second leg support frame 50 configured to support the left leg of the user.

The thigh fastener 1 or 2 may be connected to the leg support frame 50 or 55 and may fasten the leg support frame 50 or 55 to the thigh. The thigh fastening portions 1 and 2 may include a first thigh fastening portion 2 configured to fasten the first leg support frame 55 to a right thigh of the user, and a second thigh fastening portion 1 configured to fasten the second leg support frame 50 to a left thigh of the user.

In an embodiment, the first thigh fastening portion 2 may include a first cover, a first fastening frame, and a first strap, and the second thigh fastening portion 1 may include a second cover, a second fastening frame, and a second strap. The first cover and the second cover may apply torques generated by the driving modules 35 and 45 to the thighs of the user. The first cover and the second cover may be disposed on respective sides of the thighs of the user to push or pull the thighs of the user. For example, the first cover and the second cover may be arranged on the front surfaces of the thighs of the user. The first cover and the second cover may be arranged in circumferential directions of the thighs of the user. The first cover and the second cover may extend to both sides from the other end portions of the leg support frames 50 and 55 and may include curved surfaces corresponding to the thighs of the user. One ends of the first cover and the second cover may be connected to the fastening frames, and the other ends thereof may be connected to the straps.

The first fastening frame and the second fastening frame may be arranged, for example, to surround at least some portions of the circumferences of the thighs of the user, thereby preventing the thighs of the user from being separated from the leg support frames 50 and 55. The first fastening frame may have a fastening structure that connects the first cover and the first strap, and the second fastening frame may have a fastening structure that connects the second cover and the second strap.

The first strap may enclose the remaining portion of the circumference of the right thigh of the user that is not covered by the first cover and the first fastening frame, and the second strap may enclose the remaining portion of the circumference of the left thigh of the user that is not covered by the second cover and the second fastening frame. The first strap and the second strap may include, for example, an elastic material (e.g., a band).

FIGS. 5A and 5B are diagrams illustrating a configuration of a control system of a wearable device, according to an embodiment.

Referring to FIG. 5A, the wearable device 100 may be controlled by a control system 500. The control system 500 may include the control module 510, the communication module 516, the sensor module 520, a driving module 530, an input module 540, and the sound output module 550. In an embodiment, at least one (e.g., the sound output module 550) of the above components may be omitted from the control system 500, or one or more other components (e.g., a haptic module) may be added to the control system 500.

The driving module 530 may include a motor 534 configured to generate power (e.g., torque), and a motor driver circuit 532 to drive the motor 534. Although FIG. 5A illustrates the driving module 530 including one motor driver circuit 532 and one motor 534, the example of FIG. 5A is merely an example. Referring to FIG. 5B, a control system 500-1 shown in FIG. 5B may include a plurality of (e.g., two or more) motor driver circuits 532 and 532-1 and a plurality of (e.g., two or more) motors 534 and 534-1. The driving module 530 including the motor driver circuit 532 and the motor 534 may correspond to the first driving module 45 of FIG. 3, and a driving module 530-1 including the motor driver circuit 532-1 and the motor 534-1 may correspond to the second driving module 35 of FIG. 3. The following descriptions of the motor driver circuit 532 and the motor 534 may also be respectively applicable to the motor driver circuit 532-1 and the motor 534-1 illustrated in FIG. 5B.

Referring back to FIG. 5A, the sensor module 520 may include a sensor circuit including at least one sensor. The sensor module 520 may include sensor data including motion information of a user or motion information of the wearable device 100. The sensor module 520 may transmit the obtained sensor data to the control module 510. The sensor module 520 may include an IMU 522 and an angle sensor (e.g., the first angle sensor 524 and the second angle sensor 524-1) as illustrated in FIG. 5B. The IMU 522 may measure an upper body motion value of the user. For example, the IMU 522 may sense X-axis, Y-axis, and Z-axis accelerations and X-axis, Y-axis, and Z-axis angular velocities according to a motion of the user. The IMU 522 may be used to measure, for example, at least one of a forward and backward tilt, a left and right tilt, or a rotation of the body of the user. In addition, the IMU 522 may obtain motion values (e.g., acceleration values and angular velocity values) of a waist support frame (e.g., the waist support frame 20 of FIG. 3) of the wearable device. The motion values of the waist support frame 20 may correspond to upper body motion values of the user.

The angle sensor may measure a hip joint angle value according to a leg motion of the user. Sensor data that may be measured by the angle sensor may include, for example, a hip joint angle value of a right leg, a hip joint angle value of a left leg, and information on a motion direction of a leg. For example, the first angle sensor 540 of FIG. 5B may obtain the hip joint angle value of the right leg of the user, and the second angle sensor 540-1 may obtain the hip joint angle value of the left leg of the user. For example, the first angle sensor 540 and the second angle sensor 540-1 may each include, for example, an encoder and/or a hall sensor. In addition, the angle sensor may obtain a motion value of a leg support frame of the wearable device. For example, the first angle sensor 540 may obtain a motion value of the first leg support frame 55 and the second angle sensor 540-1 may obtain a motion value of the second leg support frame 50. The motion values of the leg support frames may correspond to the hip joint angle values.

In an embodiment, the sensor module 520 may further include at least one of a position sensor configured to obtain a position value of the wearable device 100, a proximity sensor configured to sense the proximity of an object, a biosignal sensor configured to detect a biosignal of the user, or a temperature sensor configured to measure an ambient temperature.

The input module 540 may receive a command or data to be used by another component (e.g., the processor 512) of the wearable device 100 from the outside (e.g., a user) of the wearable device 100. The input module 540 may include an input component circuit. The input module 540 may include, for example, a key (e.g., a button) or a touch screen.

The sound output module 550 may output a sound signal to the outside of the wearable device 100. The sound output module 550 may provide auditory feedback to the user. For example, the sound output module 550 may include a speaker configured to play back a guiding sound signal (e.g., an operation start sound, an operation error alarm, or an exercise start alarm), music content, or a guiding voice for auditorily informing predetermined information (e.g., exercise result information or exercise posture evaluation information).

In an embodiment, the control system 500 may further include a battery (not shown) configured to supply power to each component of the wearable device. The wearable device may convert the power of the battery into power suitable for an operating voltage of each component of the wearable device and supply the converted power to each component.

The driving module 530 may generate an external force to be applied to a leg of the user under the control of the control module 510. The driving module 530 may generate a torque to be applied to the legs of the user based on a control signal generated by the control module 510. The control module 510 may transmit the control signal to the motor driver circuit 532. The motor driver circuit 532 may control the operation of the motor 534 by generating a current signal (or voltage signal) corresponding to the control signal and supplying the generated current signal to the motor 534. In some cases, the current signal may not be supplied to the motor 534. When the motor 534 is supplied with the current signal and is driven, the motor 534 may generate torque for an assistance force to assist leg motion of the user or for a resistance force to impede the leg motion of the user.

The control module 510 may control the overall operation of the wearable device and may generate a control signal for controlling each component (e.g., the communication module 516 or the driving module 530). The control module 510 may include the processor 512 and a memory 514.

The processor 512 may execute, for example, software to control at least one other component (e.g., a hardware or software component) of the wearable device connected to the processor 512, and may perform a variety of data processing or computation. The software may include an application for providing a GUI. According to an embodiment, as at least a part of data processing or computation, the processor 512 may store instructions or data received from another component (e.g., the communication module 516) in the memory 514, may process the instructions or the data stored in the memory 514, and may store result data in the memory 514. According to an embodiment, the processor 512 may include a main processor (e.g., a central processing unit (CPU) or an application processor (AP)), or an auxiliary processor (e.g., a graphics processing unit (GPU), a neural processing unit (NPU), an image signal processor (ISP), a sensor hub processor, or a communication processor (CP)) that is operable independently of or in conjunction with the main processor. The auxiliary processor may be implemented separately from the main processor or as a part of the main processor.

The memory 514 may store a variety of data used by at least one component (e.g., the processor 512) of the control module 510. The variety of data may include, for example, software, sensor data, input data or output data for instructions related thereto. The memory 514 may include a volatile memory or a non-volatile memory (e.g., random-access memory (RAM), dynamic RAM (DRAM), or static RAM (SRAM)).

The communication module 516 may support establishing a direct (e.g., wired) communication channel or a wireless communication channel between the control module 510 and another component of the wearable device 100 or an external electronic device (e.g., the electronic device 210 or the other wearable device 220 of FIG. 2) and performing communication via the established communication channel. The communication module 516 may include a communication circuit configured to perform a communication function. For example, the communication module 516 may receive a control signal from an electronic device (e.g., the electronic device 210) and transmit the sensor data obtained by the sensor module 520 to the electronic device. According to an embodiment, the communication module 516 may include one or more CPs (not shown) that are operable independently of the processor 512 and that support direct (e.g., wired) communication or wireless communication. According to an embodiment, the communication module 516 may include a wireless communication module (e.g., a cellular communication module, a short-range wireless communication module, or a global navigation satellite system (GNSS) communication module), and/or a wired communication module. A corresponding one of the above communication modules may communicate with another component of the wearable device 100 and/or an external electronic device via a short-range communication network, such as BluetoothTM, Wi-Fi, or infrared data association (IrDA), or a long-range communication network, such as a legacy cellular network, a 5G network, a next-generation communication network, the Internet, or a computer network (e.g., a local area network (LAN) or a wide region network (WAN)).

In an embodiment, the control system 500, 500-1 may further include a haptic module (not shown). The haptic module may provide haptic feedback to the user under the control of the processor 512. The haptic module may convert an electrical signal into a mechanical stimulus (e.g., a vibration or a movement) or an electrical stimulus which may be recognized by a user via his or her tactile sensation or kinesthetic sensation. The haptic module may include a motor, a piezoelectric element, or an electrical stimulation device. In an embodiment, the haptic module may be positioned in at least one of the base body (e.g., the base body 80), the first thigh fastening portion 2, or the second thigh fastening portion 1.

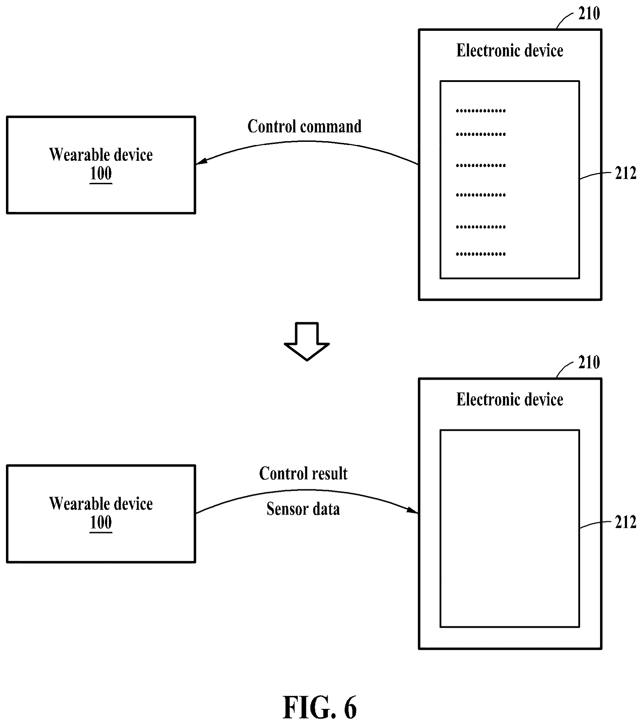

FIG. 6 is a diagram illustrating an interaction between a wearable device and an electronic device, according to an embodiment.

Referring to FIG. 6, the wearable device 100 may communicate with the electronic device 210. For example, the electronic device 210 may be a user terminal of a user who uses the wearable device 100 or a controller device dedicated to the wearable device 100. In an embodiment, the wearable device 100 and the electronic device 210 may be connected to each other through short-range wireless communication (e.g., Bluetooth communication or Wi-Fi communication).

In an embodiment, the electronic device 210 may check a state of the wearable device 100 or execute an application to control or operate the wearable device 100. A screen of a user interface (UI) may be displayed to control an operation of the wearable device 100 or determine an operation mode of the wearable device 100 on a display 212 of the electronic device 210 through the execution of the application. The UI may be, for example, a GUI.

In an embodiment, the user may input a command for controlling the operation of the wearable device 100 (e.g., an execution command to a walking assistance mode, an exercise assistance mode, or a physical ability measurement mode) or change settings of the wearable device 100 through a GUI screen on the display 212 of the electronic device 210. The electronic device 210 may generate a control command (or control signal) corresponding to an operation control command or a setting change command input by the user and transmit the generated control command to the wearable device 100. The wearable device 100 may operate according to the received control command and may transmit a control result according to the received control command and/or sensor data sensed by a sensor module of the wearable device 100 to the electronic device 210. The electronic device 210 may provide the user with result information (e.g., gait ability information, exercise ability information, or exercise motion evaluation information) derived by analyzing the control result and/or the sensor data through the GUI screen.

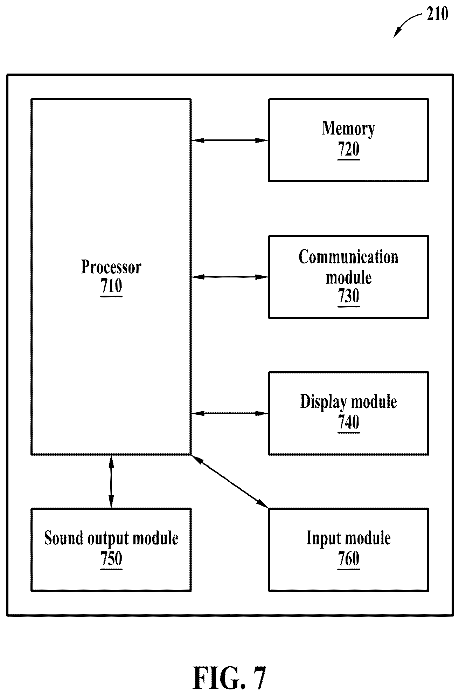

FIG. 7 is a diagram illustrating a configuration of an electronic device, according to an embodiment.

Referring to FIG. 7, the electronic device 210 may include a processor 710, a memory 720, a communication module 730, a display module 740, a sound output module 750, and an input module 760. In an embodiment, at least one (e.g., the sound output module 750) of the above components may be omitted from the electronic device 210, or one or more other components (e.g., a sensor module and a battery) may be added to the electronic device 210.

The processor 710 may control at least one other component (e.g., a hardware or software component) of the electronic device 210, and may perform a variety of data processing or computation. According to an embodiment, as at least a part of data processing or computation, the processor 710 may store instructions or data received from another component (e.g., the communication module 730) in the memory 720, process the instructions or data stored in the memory 720, and store result data in the memory 720.

In an embodiment, the processor 710 may include a main processor (e.g., a CPU or an AP) or an auxiliary processor (e.g., a GPU, an NPU, an ISP, a sensor hub processor, or a CP) that is operable independently of or in conjunction with the main processor.

The memory 720 may store a variety of data used by at least one component (e.g., the processor 710 or the communication module 730) of the electronic device 210. The data may include, for example, a program (e.g., an application), and input data or output data for a command related thereto. The memory 720 may include at least one instruction executable by the processor 710. The memory 720 may include, for example, a volatile memory or a non-volatile memory.

The communication module 730 may support establishing a direct (e.g., wired) communication channel or a wireless communication channel between the electronic device 210 and another electronic device (e.g., the wearable device 100, the other wearable device 220, or the server 230) and performing communication via the established communication channel. The communication module 730 may include a communication circuit configured to perform a communication function. The communication module 730 may include one or more CPs that are operable independently of the processor 710 (e.g., an AP) and that support direct (e.g., wired) communication or wireless communication. According to an embodiment, the communication module 290 may include a wireless communication module configured to perform wireless communication (e.g., a Bluetooth communication module, a cellular communication module, a Wi-Fi communication module, or a GNSS communication module) or a wired communication module (e.g., a LAN communication module or a power line communication (PLC) module). For example, the communication module 730 may transmit a control command to the wearable device 100 and receive, from the wearable device 100, at least one of sensor data including body motion information of the user who is wearing the wearable device 100, state data of the wearable device 100, or control result data corresponding to the control command.

The display module 740 may visually provide information to the outside (e.g., the user) of the electronic device 210. The display module 740 may include, for example, a liquid-crystal display (LCD) or organic light-emitting diode (OLED) display, a hologram device, or a projector device. The display module 740 may further include a control circuit configured to control the driving of a display. In an embodiment, the display module 740 may include a touch sensor adapted to sense a touch, or a pressure sensor adapted to measure an intensity of a force incurred by the touch.

The sound output module 750 may output a sound signal to the outside of the electronic device 210. The sound output module 750 may include a speaker configured to play back a guiding sound signal (e.g., an operation start sound or an operation error alarm), music content, or a guiding voice based on the state of the wearable device 100. For example, when it is determined that the wearable device 100 is not properly worn on the body of the user, the sound output module 750 may output a guiding voice for informing the user is wearing the wearable device 100 abnormally or for guiding the user to wear the wearable device 100 normally. The sound output module 750 may output, for example, a guiding voice corresponding to exercise evaluation information or exercise result information obtained by evaluating an exercise of the user.

The input module 760 may receive a command or data to be used by another component (e.g., the processor 710) of the electronic device 210, from the outside (e.g., the user) of the electronic device 210. The input module 760 may include an input component circuit and may receive a user input. The input module 760 may include, for example, a key (e.g., a button) or a touch screen.

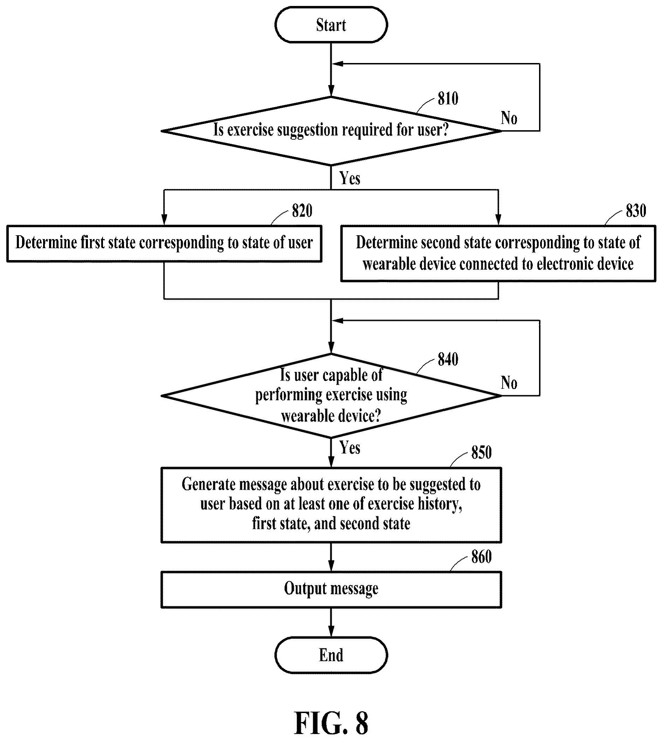

FIG. 8 is a flowchart of a message output method according to an embodiment.

Operations 810 to 860 below may be performed by an electronic device (e.g., the electronic device 210 of FIGS. 2, 6, and 7). The electronic device may include a processor (e.g., the processor 512 of FIG. 5A or the processor 710) and a communication module (e.g., the communication module 516 of FIG. 5A or the communication module 730).

According to an embodiment, operations 810 to 860 may be performed while a user of the electronic device is wearing a smartwatch (e.g., the smartwatch 224 of FIG. 2) connected to the electronic device. Hereinafter, it is described that operations 810 to 860 are performed while the user is wearing the smartwatch, however, even when the user wears another wearable device (e.g., the earphones 222 or the smart glasses 226) other than the smartwatch, operations 810 to 860 may be performed based on the other wearable device. For example, the term "smartwatch" may be replaced with the term "earphones" or "smart glasses."

In operation 810, the electronic device may determining whether an exercise suggestion is required for a user based on an exercise history of the user of the electronic device.

According to an embodiment, the electronic device may determine whether the exercise suggestion is required by comparing the exercise history and an exercise goal set for the user. For example, the electronic device may store a history that the user has exercised while wearing a wearable device (e.g., the wearable device 100 of FIGS. 1 to 4 and FIG. 6). For example, the electronic device may store a history that the user has exercised without wearing the wearable device. The electronic device may store an exercise history collected through a smartwatch worn by the user while exercising. The exercise history may include histories of the followings: an exercise type, duration, the number of times of exercise sessions, exercise timing (e.g., weekdays, weekends, or all), and/or exercise performance (e.g., calories burned, a movement distance, the number of repetitions or sets, etc.). The user may set a goal for each of the exercise type, the duration, the number of times of exercise sessions, the exercise timing, and/or the exercise performance.

According to an embodiment, the electronic device may compare the exercise history of the user during a period of time (e.g., a week) with each item of the exercise goal set for the user. For example, the electronic device may determine that the exercise suggestion is required when the history and the goal of exercise timing do not match and the history of the number of exercise sessions fails to reach the goal thereof. For example, the electronic device may determine that the exercise suggestion is required when the history and the goal of the exercise type do not match. For example, the electronic device may determine that the exercise suggestion is required when the history of the exercise performance fails to reach the goal thereof. For example, the electronic device may determine that no exercise suggestion is required when the histories and the goals of the exercise type and the exercise timing match with each other and the histories of the duration, the number of times of exercise sessions, and the exercise performance exceed the goals thereof.

According to an embodiment, the electronic device may determine that the exercise suggestion is required for the user, when a notification that an exercise buddy of the user registered in the electronic device has started exercising is received. The user may register an account or a contact of the exercise buddy in advance in association with an account or a contact of the user, and set to receive the notification when the exercise buddy starts exercising.

According to an embodiment, operations 820 and 830 below may be performed in parallel and independently.

In operation 820, the electronic device may determine a first state corresponding to a state of the user when it is determined that the exercise suggestion is required.

According to an embodiment, the electronic device may determine the first state based on at least one of a sensing result of the smartwatch or a sensing result of the electronic device. For example, the electronic device may determine whether the user wearing the smartwatch moves, and determine the duration of a movement state or a stationary state as the first state. For example, the electronic device may determine whether the user executes a specific application, such as an entertainment application, using the electronic device, and determine the duration of the execution of the application as the first state. For example, the electronic device may determine whether the user uses a home appliance and determine, as the first state, a usage time collected by an application of the Internet of Things (or smart home) installed on the electronic device.

According to an embodiment, the electronic device may determine the first state using the sensing result of the smartwatch and/or physical conditions of the user recorded in a healthcare application. For example, the electronic device may determine a body composition change result as the first state.

In operation 830, when it is determined that the exercise suggestion is required, the electronic device may determine a second state corresponding to the state of the wearable device connected to the electronic device.

For example, the second state corresponding to the state of the wearable device may be determined by a charge level (or remaining battery power) of the wearable device. For example, the second state corresponding to the state of the wearable device may indicate whether the wearable device is connected to a cradle of the wearable device.

In operation 840, the electronic device may determine whether the user is capable of performing the exercise using the wearable device based on the first state and the second state.

According to an embodiment, when the first state corresponds to a state in which the user is static for a threshold time or longer and the second state corresponds to a state of being charged at a threshold charge level or more, the electronic device may determine that the user is capable of performing the exercise using the wearable device. For example, when the first state indicates that the user has remained stationary for 10 minutes or more, the user has continued to execute the entertainment application for 30 minutes or more using the electronic device, or the user has continued to watch a television (TV) for 1 hour or more, the first state may correspond to a state in which the user has remained stationary for the threshold time or more. For example, when the remaining battery power of the wearable device is 70% or more, the second state may correspond to a state in which the wearable device is charged at the threshold charge level or more.

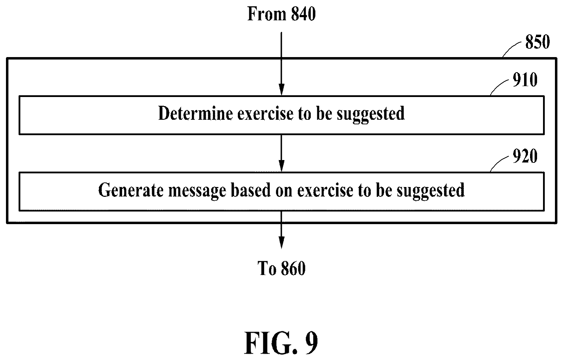

In operation 850, when the user is capable of performing the exercise using the wearable device, the electronic device may generate a message about an exercise to be suggested to the user based on at least one of the exercise history, the first state, and the second state. A method of determining the exercise to be suggested to the user and generating the message will be described in detail with reference to FIG. 9.

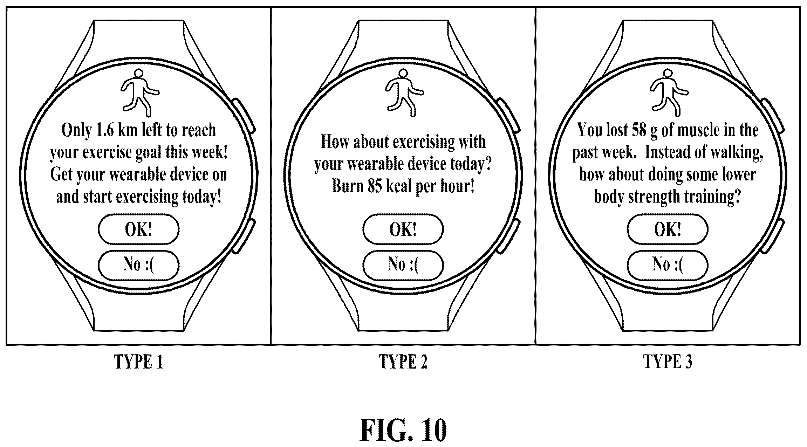

In operation 860, the electronic device may output the message. For example, the electronic device may output the message to a display (e.g., the display 212 of FIG. 6) of the electronic device. For example, the electronic device may control a smartwatch to output the message to a display of the smartwatch worn by the user. A method of outputting the message will be described in detail below with reference to FIGS. 9 and 10.

FIG. 9 is a flowchart of a method of generating a message according to an embodiment.

According to an embodiment, operation 850 described above with reference to FIG. 8 may include operations 910 and 920 to be described hereinafter. Operations 910 and 920 may be performed by an electronic device (e.g., the electronic device 210 of FIGS. 2, 6, and 7) after operation 840 of FIG. 8. The electronic device may include a processor (e.g., the processor 512 of FIG. 5A or the processor 710) and a communication module (e.g., the communication module 516 of FIG. 5A or the communication module 730).

According to an embodiment, operations 910 and 920 below may be performed while a user of the electronic device is wearing a smartwatch (e.g., the smartwatch 224 of FIG. 2) connected to the electronic device.

In operation 910, the electronic device may determine the exercise to be suggested to the user. For example, the electronic device may determine the exercise to be suggested to the user based on at least one of the exercise history of the user, the first state, and the second state.

According to an embodiment, the electronic device may determine the exercise to be suggested to achieve an exercise goal that has not achieved, among the exercise history of the user.

According to an embodiment, the electronic device may determine to suggest a recently performed exercise so that an exercise effect does not disappear when the number of times (or a frequency) of the exercise of the user is reduced.

According to an embodiment, when the exercise history of the user indicates a predetermined degree or more of inefficiency (e.g., a decrease in calories burned per hour, failure to achieve a target movement distance, and the like) when exercising without wearing the wearable device compared to exercising while wearing the wearable device, the electronic device may determine to suggest exercising while wearing the wearable device.

According to an embodiment, the electronic device may determine to suggest various exercises that may be performed while wearing the wearable device when the user performs the exercise biased to certain exercise types.

According to an embodiment, the electronic device may determine the exercise to be suggested based on the charge level of the wearable device at a time point of suggesting exercising while wearing the wearable device. For example, when the wearable device is charged below the threshold charge level and thus is not capable of executing an exercise program for some exercise types, the electronic device may determine to suggest an exercise that may be performed in a low power mode. For example, the electronic device may determine to suggest the exercise that may be performed for each section of the charge level of the wearable device. For example, the electronic device may determine to induce charging of the wearable device.

According to an embodiment, the electronic device may determine the exercise to be suggested as one of an indoor exercise or an outdoor exercise based on the exercise history of the user and current weather information. The electronic device may determine the exercise to be suggested using information on current weather conditions, a temperature, an ultraviolet (UV) level, precipitation, or season. The electronic device may determine to suggest doing a home exercise or an outdoor exercise while wearing the wearable device.

According to an embodiment, the electronic device may determine the exercise to be suggested based on the exercise history of the user and a set exercise mode. The user may set an exercise curriculum provided by a specific instructor or trainer. The electronic device may determine to suggest exercising while wearing the wearable device based on the exercise history of the user and the set exercise mode.

In operation 920, the electronic device may generate a message based on the exercise to be suggested.

FIG. 10 is a diagram illustrating types of message outputs according to an embodiment.

According to an embodiment, an electronic device (e.g., the electronic device 210 of FIGS. 2, 6, and 7) may control a smartwatch (e.g., the smartwatch 224 of FIG. 2) worn by a user to output a message to a display of the smartwatch.

As described above for operation 850 of FIG. 8, the electronic device may generate a message about the exercise to be suggested to the user based on at least one of the exercise history of the user, the first state, and the second state. As illustrated in FIG. 10, the smartwatch may output different types of messages about the exercise to be suggested to the user.

According to an embodiment, in TYPE 1, the smartwatch may output a message about the exercise to be suggested to the user, generated based on the exercise history of the user. For example, the electronic device may generate the message for achieving the exercise goal that has not achieved among the exercise history of the user.

According to an embodiment, in TYPE 2, the smartwatch may output a message that suggests exercising while wearing the wearable device, generated based on the exercise history of the user. For example, when the exercise history of the user indicates a predetermined degree or more of inefficiency (e.g., a decrease in calories burned per hour, failure to achieve a target movement distance, and the like) when exercising without wearing the wearable device compared to exercising while wearing the wearable device, the electronic device may output a message that suggests exercising while wearing the wearable device.

According to an embodiment, in TYPE 3, the smartwatch may output a message generated based on the first state. For example, the electronic device may generate a message that suggests exercising to improve the physical conditions of the user based on changes in the body composition of the user.

According to an embodiment, the smartwatch may output a message generated based on the second state. For example, when the wearable device is charged below the threshold charge level and thus is not capable of executing an exercise program for some exercise types, the electronic device may generate a message that suggests an exercise that may be performed in a low power mode. For example, the electronic device may generate a message that suggests the exercise that may be performed for each section of the charge level of the wearable device.

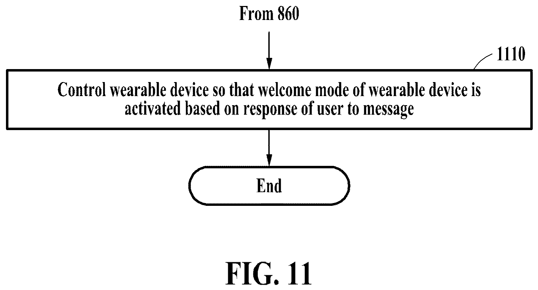

FIG. 11 is a flowchart of a method of controlling a wearable device according to an embodiment.

According to an embodiment, operation 1110 described below may be performed by an electronic device (e.g., the electronic device 210 of FIGS. 2, 6, and 7) after operation 860 of FIG. 8. The electronic device may include a processor (e.g., the processor 512 of FIG. 5A or the processor 710) and a communication module (e.g., the communication module 516 of FIG. 5A or the communication module 730).

According to an embodiment, operation 1110 may be performed while a user of the electronic device is wearing a smartwatch (e.g., the smartwatch 224 of FIG. 2) connected to the electronic device.

In operation 1110, the electronic device may control a wearable device (e.g., the wearable device 100 of FIGS. 1 to 4 and FIG. 6) to activate a welcome mode of the wearable device based on a response of the user to the message. For example, when the user responds with acceptance to a message that suggests an exercise output to the smartwatch, the welcome mode of the wearable device may be activated. For example, when the user responds with rejection to the message that suggests the exercise output to the smartwatch, the smartwatch may ask the user why the user rejects to exercise and output a message that suggests exercising again after a predetermined period of time.

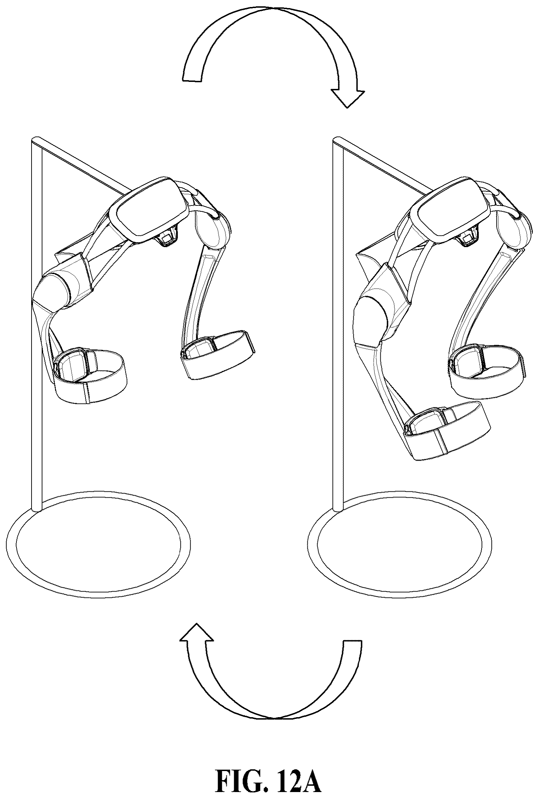

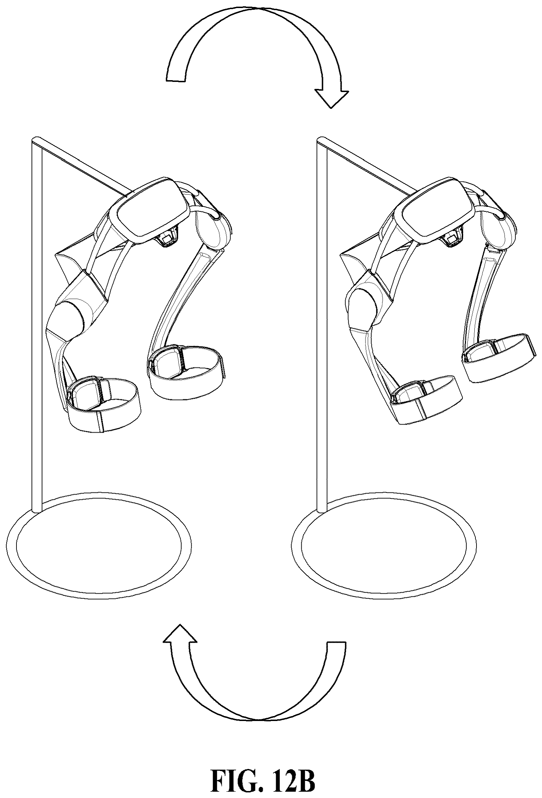

According to an embodiment, the wearable device may control left and right leg support frames (e.g., the leg support frames 50 and 55 of FIG. 3) to move to correspond to the exercise to be suggested when the welcome mode is activated. FIGS. 12A and 12B are diagrams illustrating a welcome mode of a wearable device according to an embodiment.

According to an embodiment, referring to FIG. 12A, when the exercise to be suggested to the user is a step exercise, the wearable device may control the left and right leg support frames to move in different directions when the welcome mode is activated. A step exercise may include cardio exercises such as interval walking, power walking, walking in water, and knee-ups. For example, the wearable device may be controlled so that a first driving module and a second driving module (e.g., the first driving module 45 and the second driving module 35 of FIG. 3) alternately rotate ±15 degrees in different directions based on the state in which the wearable device is placed on the cradle.

According to an embodiment, referring to FIG. 12B, when the exercise to be suggested to the user is a non-step exercise, the wearable device may control the left and right leg support frames to move in the same direction when the welcome mode is activated. A non-step exercise may include strength training exercises such as deep squats, half squats, and kickbacks. For example, the wearable device may be controlled so that the first driving module and the second driving module rotate ± 15 degrees in the same direction based on the state in which the wearable device is placed on the cradle.

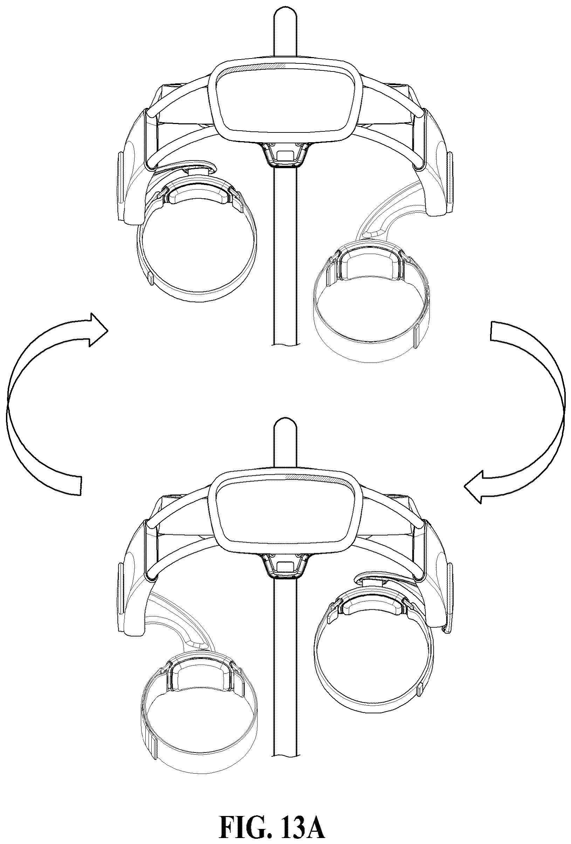

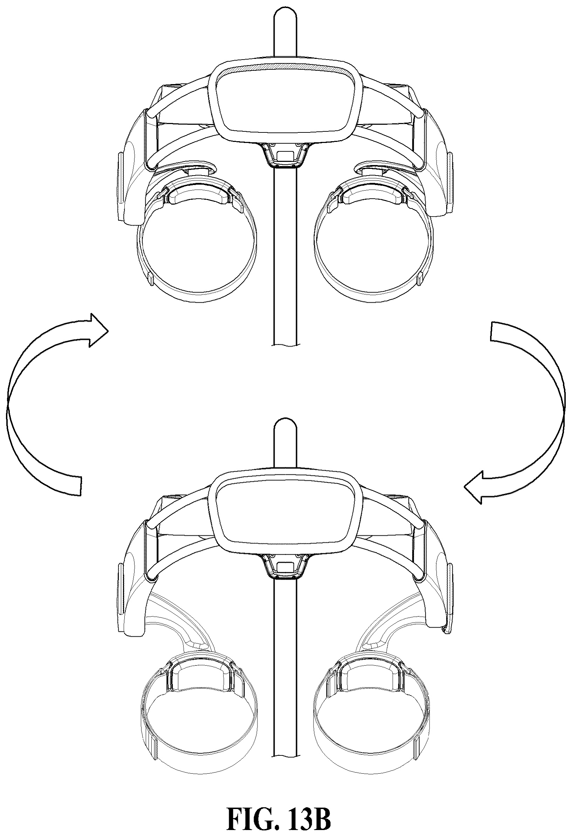

According to an embodiment, a lighting unit (e.g., the lighting unit 85 of FIG. 3) of the wearable device may be controlled to blink in response to the exercise to be suggested, when the welcome mode is activated. FIGS. 13A and 13B are diagrams illustrating a welcome mode of a wearable device according to an embodiment.

According to an embodiment, referring to FIG. 13A, in case where the exercise to be suggested to the user is the step exercise, the lighting unit of the wearable device may be controlled to blink alternately on left and right sides when the welcome mode is activated.

According to an embodiment, referring to FIG. 13B, when the exercise to be suggested to the user is the non-step exercise, the lighting unit of the wearable device may be controlled to blink simultaneously on both the left and right sides when the welcome mode is activated.

According to an embodiment, the wearable device may be controlled so that a welcome sound corresponding to the exercise to be suggested is output through a sound output module (e.g., the sound output module 550 of FIGS. 5A and 5B) when the welcome mode is activated. For example, when the exercise to be suggested to the user is the step exercise, the wearable device may output a sound that is played at a relatively higher pitch and faster beat than in case where the exercise to be suggested is the non-step exercise. For example, the welcome sound may be played in a beat synchronized with the blinking of the lighting unit.

According to an embodiment, the wearable device may deactivate the welcome mode when a distance between the user and the wearable device is less than a threshold distance. The wearable device may determine whether the user is wearing the wearable device. The wearable device may transmit wearing information indicating that the user is wearing the wearable device to the electronic device when the user is wearing the wearable device.

According to an embodiment, the electronic device may receive the wearing information indicating that the user is wearing the wearable device from the wearable device. The electronic device may transmit the wearing information to a smartwatch. The smartwatch may display various screens based on sensing information from sensors included in the smartwatch and/or information or signals received from electronic device. For example, the smartwatch may display messages or graphic effects corresponding to a state in which the user moves to wear the wearable device, a state in which the user removes the wearable device from a cradle and wears it on, or a state in which the user is wearing the wearable device.

According to an embodiment, the electronic device may set an exercise program for exercise when the wearing information is received. The user may check real-time performance results for the set exercise program through the electronic device and/or the smartwatch. The electronic device may store and manage the results of the exercise performed by the user using the exercise program as the exercise history.

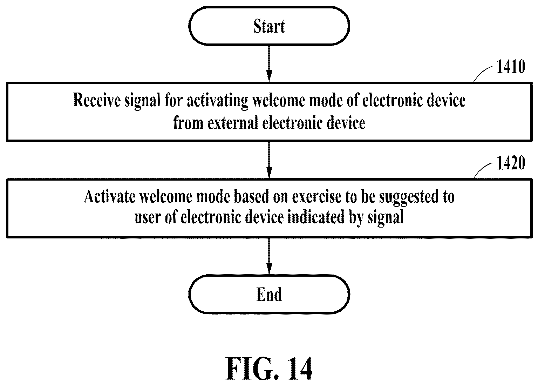

FIG. 14 is a flowchart of a method of activating a welcome mode of a wearable device according to an embodiment.

According to an embodiment, operations 1410 and 1420 described below may be performed by an electronic device (e.g., the wearable device 100 of FIGS. 1 to 4 and FIG. 6).

In operation 1410, the electronic device may receive a signal for activating a welcome mode of the electronic device from an external electronic device (e.g., the electronic device 210 of FIGS. 2, 6, and 7) connected to the electronic device through the communication module.

In operation 1420, the electronic device may activate the welcome mode based on the exercise to be suggested to the user of the electronic device indicated by the signal.

According to an embodiment, the electronic device may activate the welcome mode by controlling at least one of left and right leg support frames (e.g., the leg support frames 50 and 55 of FIG. 3), a lighting unit (e.g., the lighting unit 85 of FIG. 3), or a sound output module (e.g., the sound output module 550 of FIGS. 5A and 5B) of the electronic device based on the exercise to be suggested to the user. The welcome mode has been described in detail with reference to FIGS. 11, 12A, 12B, 13A, and 13B, and thus, any duplicate description will be omitted.

According to an embodiment, the electronic device may deactivate the welcome mode when a distance between the user and the electronic device is less than a threshold distance. The distance between the user and the electronic device may represent a distance between a smartwatch worn by the user (e.g., the smartwatch 224 of FIG. 2) and the electronic device.

According to an embodiment, the electronic device may determine whether the user is wearing the electronic device (or whether the electronic device has been worn by the user). For example, the electronic device may include means for determining whether a waist fastening portion (e.g., the waist fastening portion 60 of FIGS. 3 and 4) has fully secured a waist support frame (e.g., the waist support frame 20 of FIGS. 3 and 4) to the waist of the user, and whether thigh fastening portions (e.g., the thigh fastening portions 1 and 2 of FIG. 3) have fully secured leg support frames (e.g., the leg support frames 50 and 55 of FIG. 3) to the thighs of the user. For example, the electronic device may determine whether the belts of the waist fastening portion and the thigh fastening portions are fully fastened.

According to an embodiment, the electronic device may transmit wearing information indicating that the user is wearing the electronic device to an external electronic device when the user is wearing the electronic device.

According to an embodiment, a message output method performed by an electronic device 210 may include determining 810 whether an exercise suggestion is required for a user based on an exercise history of the user of the electronic device 210, when it is determined that the exercise suggestion is required, determining 820 a first state corresponding to a state of the user, when it is determined that the exercise suggestion is required, determining 830 a second state corresponding to a state of a wearable device 100 connected to the electronic device, determining 840 whether the user is capable of performing an exercise using the wearable device 100 based on the first state and the second state, when the user is capable of performing the exercise using the wearable device 100, generating 850 a message about an exercise to be suggested to the user based on at least one of the exercise history, the first state, and the second state, and outputting 860 the message.

According to an embodiment, the message output method may further include controlling 1110 the wearable device 100 to activate a welcome mode of the wearable device 100 based on a response of the user to the message.

According to an embodiment, in a case where the exercise to be suggested is a step exercise, when the welcome mode is activated, the wearable device 100 may be controlled to move left and right leg support frames 50 and 55 in different directions.

According to an embodiment, in a case where the exercise to be suggested is a non-step exercise, when the welcome mode is activated, the wearable device 100 may be controlled to move the left and right leg support frames 50 and 55 in the same direction.

According to an embodiment, when the welcome mode is activated, a lighting unit 85 of the wearable device 100 may be controlled to blink in response to the exercise to be suggested.

According to an embodiment, when the welcome mode is activated, a welcome sound corresponding to the exercise to be suggested may be output by the wearable device 100.

According to an embodiment, the determining 810 of whether the exercise suggestion is required based on the exercise history of the user of the electronic device 210 may include determining whether the exercise suggestion is required by comparing the exercise history and an exercise goal set for the user.

According to an embodiment, the determining 840 of whether the user is capable of performing the exercise using the wearable device 100 based on the first state and the second state may include, when the first state corresponds to a state in which the user is static for a threshold time or longer and the second state corresponds to a state of being charged to a threshold charge level or more, determining that the user is capable of performing the exercise using the wearable device 100.

According to an embodiment, the message output method may further include, when a notification that an exercise buddy of the user registered in the electronic device 210 has started exercising is received, determining that the exercise suggestion is required for the user.

According to an embodiment, the generating 850 of the message about the exercise to be suggested to the user may include determining 910 the exercise to be suggested, and generating 920 the message based on the exercise to be suggested.

According to an embodiment, the determining 910 of the exercise to be suggested may include determining the exercise to be suggested as one of an indoor exercise or an outdoor exercise based on the exercise history and current weather information.

According to an embodiment, the determining 910 of the exercise to be suggested may include determining the exercise to be suggested based on the exercise history and a set exercise mode.

According to an embodiment, the message output method may include receiving wearing information indicating that the user is wearing the wearable device 100 from the wearable device 100, and when the wearing information is received, setting an exercise program for the exercise.

According to an embodiment, an electronic device may include a communication module 516 that exchanges data with an external device, at least one processor 512 connected to the communication module 516, and memory 514 storing instructions executable by the at least one processor 512, and the instructions, when executed by the at least one processor 512, may cause the electronic device 100 to perform receiving 1410 a signal for activating a welcome mode of the electronic device 100 from an external electronic device 210 connected to the electronic device 100 through the communication module 516, and activating 1420 the welcome mode by controlling at least one of left and right leg support frames 50 and 55, a lighting unit 85, or a sound output module 550 of the electronic device 100 based on an exercise to be suggested to the user of the electronic device 100 indicated by the signal.

According to an embodiment, in a case where the exercise to be suggested is a step exercise, when the welcome mode is activated, the left and right leg support frames 50 and 55 of the electronic device 100 may be controlled to move in different directions.

According to an embodiment, in a case where the exercise to be suggested is a non-step exercise, when the welcome mode is activated, the left and right leg support frames 50 and 55 of the electronic device 100 may be controlled to move in the same direction.

According to an embodiment, when the welcome mode is activated, the lighting unit 85 of the electronic device 100 may be controlled to blink in response to the exercise to be suggested.

According to an embodiment, when the welcome mode is activated, a welcome sound corresponding to the exercise to be suggested is output by the electronic device 100.

According to an embodiment, the instructions, when executed by the at least one processor 512, may further cause the electronic device 100 to perform, when a distance between the user and the electronic device 100 is less than a threshold distance, deactivating the welcome mode, determining whether the user is wearing the electronic device 100, and when the user is wearing the electronic device 100, transmitting wearing information indicating that the user is wearing the electronic device 100 to the external electronic device 210.

According to an embodiment, an electronic device 210 may include a communication module 730 that exchanges data with an external device, at least one processor 710 connected to the communication module, and memory 720 storing instructions executable by the at least one processor 710, and the instructions, when executed by the at least one processor 710, may cause the electronic device 210 to perform determining 810 whether an exercise suggestion is required for a user based on an exercise history of the user of the electronic device 210, when it is determined that the exercise suggestion is required, determining 820 a first state corresponding to a state of the user, when it is determined that the exercise suggestion is required, determining 830 a second state corresponding to a state of a wearable device 100 connected to the electronic device 210, determining 840 whether the user is capable of performing an exercise using the wearable device 100 based on the first state and the second state, when the user is capable of performing the exercise using the wearable device 100, generating 850 a message about an exercise to be suggested to the user based on at least one of the exercise history, the first state, and the second state, and outputting 860 the message.

The embodiments described herein may be implemented using a hardware component, a software component and/or a combination thereof. A processing device may be implemented using one or more general-purpose or special-purpose computers, such as, for example, a processor, a controller and an arithmetic logic unit (ALU), a DSP, a microcomputer, a FPGA, a programmable logic unit (PLU), a microprocessor or any other device capable of responding to and executing instructions in a defined manner. The processing device may run an OS and one or more software applications that run on the OS. The processing device also may access, store, manipulate, process, and generate data in response to execution of the software. For purpose of simplicity, the description of a processing device is used as singular; however, one skilled in the art will appreciate that a processing device may include multiple processing elements and/or multiple types of processing elements. For example, the processing device may include a plurality of processors, or a single processor and a single controller. In addition, different processing configurations are possible, such as parallel processors.

The software may include a computer program, a piece of code, an instruction, or some combinations thereof, to independently or collectively instruct or configure the processing device to operate as desired. Software and/or data may be embodied permanently or temporarily in any type of machine, component, physical or virtual equipment, computer storage medium or device, or in a propagated signal wave capable of providing instructions or data to or being interpreted by the processing device. The software may also be distributed over network-coupled computer systems so that the software is stored and executed in a distributed fashion. The software and data may be stored by one or more non-transitory computer-readable recording mediums.

The methods according to the above-described embodiments may be recorded in non-transitory computer-readable media including program instructions to implement various operations of the above-described embodiments. The media may also include, alone or in combination with the program instructions, data files, data structures, and the like. The program instructions recorded on the media may be those specially designed and constructed for the purposes of embodiments, or they may be of the kind well-known and available to those having skill in the computer software arts. Examples of non-transitory computer-readable media include magnetic media such as hard disks, floppy disks, and magnetic tape; optical media such as CD-ROM discs and/or DVDs; magneto-optical media such as optical discs; and hardware devices that are specially configured to store and perform program instructions, such as read-only memory (ROM), random access memory (RAM), flash memory, and the like. Examples of program instructions include both machine code, such as produced by a compiler, and files containing higher-level code that may be executed by the computer using an interpreter.

The above-described devices may be configured to act as one or more software modules in order to perform the operations of the above-described embodiments, or vice versa.

As described above, although the embodiments have been described with reference to the limited drawings, a person skilled in the art may apply various technical modifications and variations based thereon. For example, suitable results may be achieved if the described techniques are performed in a different order and/or if components in a described system, architecture, device, or circuit are combined in a different manner and/or replaced or supplemented by other components or their equivalents.

Therefore, other implementations, other embodiments, and equivalents to the claims are also within the scope of the following claims.

## Claims

1. A message output method performed by an electronic device 210, the message output method comprising:
determining 810 whether an exercise suggestion is required for a user based on an exercise history of the user of the electronic device 210;
when it is determined that the exercise suggestion is required, determining 820 a first state corresponding to a state of the user;
when it is determined that the exercise suggestion is required, determining 830 a second state corresponding to a state of a wearable device 100 connected to the electronic device;
determining 840 whether the user is capable of performing an exercise using the wearable device 100 based on the first state and the second state;
when the user is capable of performing the exercise using the wearable device 100, generating 850 a message about an exercise to be suggested to the user based on at least one of the exercise history, the first state, and the second state; and
outputting 860 the message.

2. The message output method of claim 1, further comprising:
controlling 1110 the wearable device 100 to activate a welcome mode of the wearable device 100 based on a response of the user to the message.

3. The message output method of any one of claims 1 and 2, wherein
in a case where the exercise to be suggested is a step exercise, when the welcome mode is activated, left and right leg support frames 50 and 55 of the wearable device 100 are controlled to move in different directions, and
in a case where the exercise to be suggested is a non-step exercise, when the welcome mode is activated, the left and right leg support frames 50 and 55 of the wearable device 100 are controlled to move in the same direction.

4. The message output method of any one of claims 1 to 3, wherein, when the welcome mode is activated, a lighting unit 85 of the wearable device 100 is controlled to blink in response to the exercise to be suggested.

5. The message output method of any one of claims 1 to 4, wherein, when the welcome mode is activated, a welcome sound corresponding to the exercise to be suggested is output by the wearable device 100.

6. The message output method of any one of claims 1 to 5, wherein the determining 810 of whether the exercise suggestion is required based on the exercise history of the user of the electronic device 210 comprises determining whether the exercise suggestion is required by comparing the exercise history and an exercise goal set for the user.

7. The message output method of any one of claims 1 to 6, wherein the determining 840 of whether the user is capable of performing the exercise using the wearable device 100 based on the first state and the second state comprises, when the first state corresponds to a state in which the user is static for a threshold time or longer and the second state corresponds to a state of being charged to a threshold charge level or more, determining that the user is capable of performing the exercise using the wearable device 100.

8. The message output method of any one of claims 1 to 7, further comprising:
when a notification that an exercise buddy of the user registered in the electronic device 210 has started exercising is received, determining that the
exercise suggestion is required for the user.

9. The message output method of any one of claims 1 to 8, wherein the generating 850 of the message about the exercise to be suggested to the user comprises:
determining 910 the exercise to be suggested; and
generating 920 the message based on the exercise to be suggested, and
wherein the determining 910 of the exercise to be suggested comprises determining the exercise to be suggested as one of an indoor exercise or an outdoor exercise based on the exercise history and current weather information,
or determining the exercise to be suggested based on the exercise history and a set exercise mode.

10. The message output method of any one of claims 1 to 9, further comprising:
receiving wearing information indicating that the user is wearing the wearable device 100 from the wearable device 100; and
when the wearing information is received, setting an exercise program for the exercise.

11. An electronic device 100 comprising:
a communication module 516 configured to exchange data with an external device;
at least one processor 512 connected to the communication module 516; and
memory 514 storing instructions executable by the at least one processor 512, wherein the instructions, when executed by the at least one processor 512,
cause the electronic device 100 to perform:
receiving 1410 a signal for activating a welcome mode of the electronic device 100 from an external electronic device 210 connected to the electronic device 100 through the communication module 516; and
activating 1420 the welcome mode by controlling at least one of left and right leg support frames 50 and 55, a lighting unit 85, or a sound output module 550 of the electronic device 100 based on an exercise to be suggested to the user of the electronic device 100 indicated by the signal.

12. The electronic device 100 of claim 11, wherein in a case where the exercise to be suggested is a step exercise, when the welcome mode is activated, the left and right leg support frames 50 and 55 of the electronic device 100 are controlled to move in different directions, and in a case where the exercise to be suggested is a non-step exercise, when the welcome mode is activated, the left and right leg support frames 50 and 55 of the electronic device 100 are controlled to move in the same direction.

13. The electronic device 100 of any one of claims 11 and 12, wherein, when the welcome mode is activated, the lighting unit 85 of the electronic device 100 is controlled to blink in response to the exercise to be suggested.

14. The electronic device 100 of any one of claims 11 to 13, wherein, when the welcome mode is activated, a welcome sound corresponding to the exercise to be suggested is output by the electronic device 100.

15. The electronic device 100 of any one of claims 11 to 14, wherein the instructions, when executed by the at least one processor 512, further cause the electronic device 100 to perform:
when a distance between the user and the electronic device 100 is less than a threshold distance, deactivating the welcome mode;
determining whether the user is wearing the electronic device 100; and
when the user is wearing the electronic device 100, transmitting wearing information indicating that the user is wearing the electronic device 100 to the external electronic device 210.
